(19) Europäisches Patentamt / European Patent Office / Office européen des brevets

(11) **EP 3 475 725 B1**

(12) **EUROPEAN PATENT SPECIFICATION**

(45) Date of publication and mention of the grant of the patent:
**10.06.2020 Bulletin 2020/24**

(51) Int Cl.:
**G01S 7/41** (2006.01)

(21) Application number: **17739788.2**

(22) Date of filing: **27.06.2017**

(86) International application number:
**PCT/US2017/039510**

(87) International publication number:
**WO 2018/005498 (04.01.2018 Gazette 2018/01)**

(54) **SYSTEMS AND METHODS FOR INTERPOLATION IN SYSTEMS WITH NON-LINEAR QUANTIZATION**

SYSTEME UND VERFAHREN ZUR INTERPOLATION IN SYSTEMEN MIT NICHTLINEARER QUANTISIERUNG

SYSTÈMES ET PROCÉDÉS D'INTERPOLATION DANS DES SYSTÈMES À QUANTIFICATION NON LINÉAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2016 US 201662355686 P**

(43) Date of publication of application:
**01.05.2019 Bulletin 2019/18**

(73) Proprietor: **Raytheon Company Waltham, MA 02451-1449 (US)**

(72) Inventors:
• **WOOD, Thomas E.
  Waltham, MA 02451-1449 (US)**
• **WURZBACH, James A.
  Waltham, MA 02451-1449 (US)**

(74) Representative: **Jackson, Richard Eric Carpmaels & Ransford LLP One Southampton Row London WC1B 5HA (GB)**

(56) References cited:
**WO-A1-2015/134551    US-A1- 2008 018 525**

• **Winchester, Thomas: "A time domain simulation of the pulsed radar return from a chaff cloud", Director Electronic Research Laboratory, 31 May 1992 (1992-05-31), pages i-44, XP002774039, Salisbury, South Australia, 5108 Retrieved from the Internet: URL:http://dspace.dsto.defence.gov.au/dspace/handle/1947/9024 [retrieved on 2017-09-22]**
• **LYZENGA DAVID R ET AL: "A Simple Model for Marine Radar Images of the Ocean Surface", IEEE GEOSCIENCE AND REMOTE SENSING LETTERS, IEEE SERVICE CENTER, NEW YORK, NY, US, vol. 12, no. 12, 1 December 2015 (2015-12-01), pages 2389-2392, XP011589759, ISSN: 1545-598X, DOI: 10.1109/LGRS.2015.2478390 [retrieved on 2015-11-11]**
• **Scot G. Frank ET AL: "Profiling Breast Cancer using Real-Time Quantitative PCR" In: "Rapid Cycle Real-Time PCR - Methods and Applications - Quantification", 1 January 2004 (2004-01-01), Springer, Berlin, Heidelberg, XP055443488, ISBN: 978-3-642-62317-2 pages 95-105, DOI: 10.1007/978-3-642-18840-4_10, figure 2**
• **TICHOPAD A ET AL: "Standardized determination of real-time PCR efficiency from a single reaction set-up", NUCLEIC ACIDS RESEARCH, INFORMATION RETRIEVAL LTD, GB, vol. 31, no. 20, 15 October 2003 (2003-10-15), page e122, XP002335812, ISSN: 0305-1048, DOI: 10.1093/NAR/GNG122**

• R. G. RUTLEDGE: "Sigmoidal curve-fitting redefines quantitative real-time PCR with the prospective of developing automated high-throughput applications", NUCLEIC ACIDS RESEARCH, vol. 32, no. 22, 14 December 2004 (2004-12-14), pages e178-e178, XP055042716, ISSN: 0305-1048, DOI: 10.1093/nar/gnh177

**Description**

GOVERNMENT LICENSE RIGHTS

This invention was made with government support under Sub-award 56441140 Grant No. W81XWH-14-2-0192 awarded by the United States Army. The U.S. government has certain rights in this invention.

RELATED APPLICATIONS

This application claims priority under 35 U.S.C. § 119(e) to U.S. Provisional Application Serial No. 62/355,686, titled "METHOD OF INTERPOLATION IN SYSTEMS WITH NON-LINEAR QUANTIZATION," filed on June 28, 2016.

BACKGROUND

In various systems, non-linear quantization has an adverse effect on state estimation. For example, in many systems quantization becomes rapidly (e.g., geometrically) coarser until the system reaches a saturation point. One example of this phenomenon occurs in systems that utilize Polymerase Chain Reaction (PCR) results over dozens of integer-valued PCR cycles. PCR is a laboratory technique used to amplify a limited and unknown initial amount of a marker in a patient sample. Some techniques seek to link patient conditions, such as Traumatic Brain Injury, to an estimated initial amount of the marker after a plurality of PCR cycles are evaluated.
Similar adverse effects are experienced by some radar systems. Radar Cross Section (RCS) is a measurement of the ability of a target to reflect radar signals. Often radar antennas that are calibrated to measure RCS are equipped with a log amplifier and one or more digital-to-analog converters that quantize received radar signals. In these systems, measurements performed by the antenna are quantized at the radar antenna re-visit rate. Often the radar signal range measurements rise rapidly as the target approaches the radar system, and accordingly, the response of the log amplifier grows coarser as the received signals increase in amplitude. A time domain simulation of the pulsed radar return from a chaff cloud is known from: Winchester, Thomas, "A time domain simulation of the pulsed radar return from a chaff cloud", Director Electronic Research Laboratory, Salisbury, South Australia, 5108.

SUMMARY OF THE INVENTION

In accordance with the present invention, there is provided a method as defined by claim 1.

BRIEF DESCRIPTION OF THE DRAWINGS

Various aspects of at least one embodiment are discussed below with reference to the accompanying figures, which are not intended to be drawn to scale. The figures are included to provide illustration and a further understanding of the various aspects and embodiments, and are incorporated in and constitute a part of this specification, but are not intended as a definition of the limits of the invention. In the figures, each identical or nearly identical component that is illustrated in various figures is represented by a like numeral. For purposes of clarity, not every component may be labeled in every figure. In the figures:

FIG. 1 is a radar antenna system according to various examples described herein;
FIG. 2 is a process flow for detecting a Radar Cross Section (RCS), according to various examples described herein;
FIG. 3 illustrates example plots of a model curve and a calibrated curve for a method of detecting a Radar Cross Section (RCS), according to various examples described herein;
FIG. 4 is a patient injury diagnostic system according to various examples described herein;
FIG. 5 is a process flow for detecting a patient injury or the severity of a patient injury, according to various examples described herein;
FIG. 6 illustrates example plots of a model curve and a calibrated curve for a method of detecting a patient injury or the severity of a patient injury, according to various examples described herein; and
FIG. 7 is a block diagram of a distributed computer system, according to various examples described herein.

DETAILED DESCRIPTION

Aspects and examples are generally directed to methods and systems for interpolation in systems that execute non-linear quantization routines. In particular, examples of the methods described herein may interpolate measurements in systems that utilize exponentially (e.g., geometrically) widening quantization. Particular examples described herein are directed to a method of detecting Radar Cross Section (RCS), and a method of detecting patient injuries, based on a shift between interpolated measured data and a calibrated curve. Accordingly, various aspects and examples may be used as one or more preliminary steps in medical diagnostic decisions (e.g., for Traumatic Brain Injury (TBI)), or for measuring the detectability of an aircraft or other vehicle.

As discussed above, in many systems, non-linear quantization can have an adverse effect on state estimation. In RCS estimation, the exponential separation in measurements of target range results in high uncertainty in a determination of target RCS. Similarly, in a system relying on Polymerase Chain Reaction (PCR), geometrical growth in numbers of target molecules from an unknown initial concentration in a patient sample makes estimation of the initial concentration amount challenging.

Inaccuracies in RCS estimation are typically addressed by comparing measured signal levels to a known target at a fixed range, and averaging repeated measurements to "interpolate" the quantization through the effects of randomness. Such a highly restrictive procedure is impractical (e.g., expensive and time-consuming) to implement. Systems that use PCR typically measure the initial concentration of a target based on a measurement of a correlated fluorescence. Fluorescence measurements start in a noise region and rise rapidly at each cycle of the PCR until a saturation limit is reached. To estimate the initial concentration amount, various approaches observe the few cycles between the noise limit and the saturation limit that fit a linear projection, and use linear regression to estimate the initial target concentration. That is, estimation of an initial target concentration is typically performed by calibrating the system sensitivity to the target, and using linear regression to estimate the (fractional) cycle number where the measurements would have equaled the sensitivity level. Based on the estimated initial concentration of the target, various techniques exist for correlating the initial concentration to patient trauma. While these techniques offer one approach for correlating the concentration of a target molecule with a patient injury, these approaches introduce noise, are inaccurate, and rely only on a few measurements from a large set of measured data.

As further discussed below, various examples described herein improve the executional efficiency of radar antenna systems and patient injury diagnostic systems and offer improvements in the fields of radar detection and patient injury diagnosis. According to various examples, the described methods fit a model (e.g., a model curve) to an entire time history of measurements performed by a radar antenna system or a PCR-based patient injury diagnostic system, and measure a shift between the model and a calibrated curve from a same family of functions. Calibrated curves may be generated from a plurality of sets of premeasured data mapped to an ideal curve, such as blood markers from a known population or data of a known aircraft RCS. In a system for PCR, this may include data for healthy individuals of similar characteristics, and in a system for radar antennas, this may include averages over many runs of targets having a known RCS.

As further described below, in various examples the model curve and the calibrated curve are based on the same mathematical ideal model that includes a single variable that "shifts" the model. In PCR, the single variable may include PCR cycle numbers (fractional), and in radar the single variable may include range. When the model curve is compared to the calibrated curve, the relative "shift" between functions may be used for decision making. Such aspects and examples are in contrast to the typical approaches discussed above, which directly reduce measurements to actual target values (numbers of target molecules, or RCS). As also further described, the determined "shift" uses all collected data and therefore may improve the fidelity of the described system and methods when compared to current techniques.

It is to be appreciated that embodiments of the systems and apparatuses discussed herein are not limited in application to the details of construction and the arrangement of components set forth in the following description or illustrated in the accompanying drawings. The systems and apparatuses are capable of implementation in other embodiments and of being practiced or of being carried out in various ways. Examples of specific implementations are provided herein for illustrative purposes only and are not intended to be limiting. Also, the phraseology and terminology used herein is for the purpose of description and should not be regarded as limiting. The use herein of "including," "comprising," "having," "containing," "involving," and variations thereof is meant to encompass the items listed thereafter and equivalents thereof as well as additional items. References to "or" may be construed as inclusive so that any terms described using "or" may indicate any of a single, more than one, and all of the described terms. Any references to front and back, left and right, top and bottom, upper and lower, and vertical and horizontal are intended for convenience of description, not to limit the present systems and methods or their components to any one positional or spatial orientation.

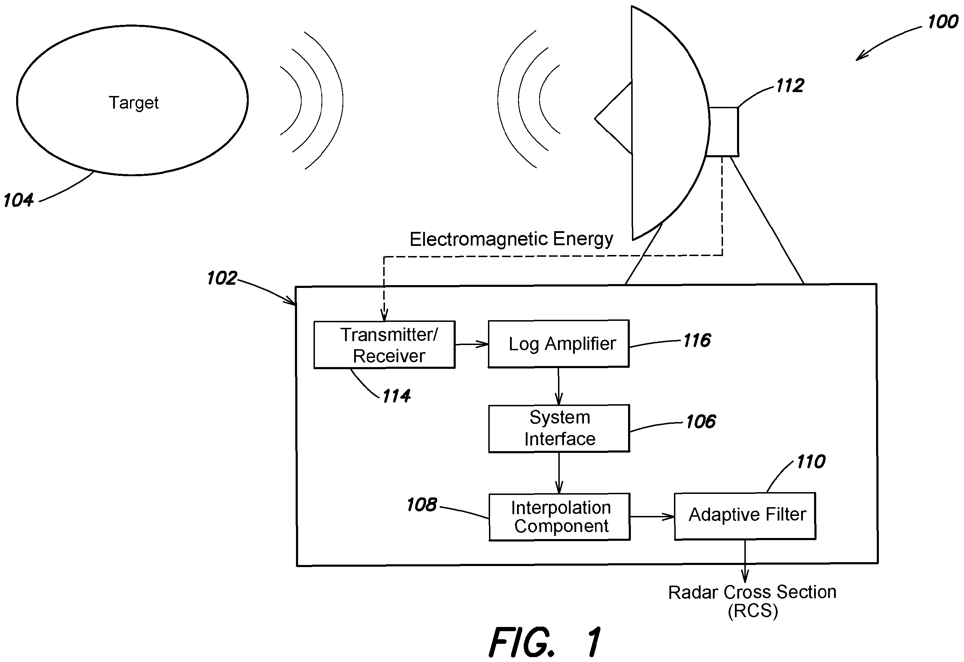

FIG. 1 illustrates one example of a radar antenna system 100 integrated with a radar antenna base station 102 to detect a Radar Cross Section (RCS) of a target 104, such as a target aircraft, using the methods described herein. As illustrated in FIG. 1, among other components, the radar antenna system 100 may include a system interface 106, an interpolation component 108, and an adaptive filter 110. As illustrated, the radar antenna system 100 is integrated within a radar antenna base station 102 that includes an antenna 112, a transceiver 114, transmitter/receiver electronics (not shown), and a log amplifier 116, among various other components. However, in various other examples the radar antenna system 100 may remotely communicate with the radar antenna base station 102 (e.g., via the system interface 106) and may be located remotely from the base station 102.

The antenna 112 emits continuous or pulsed electromagnetic energy generated by the transceiver 114. The electromagnetic energy is radiated by the antenna 112 along a transmit path in a direction of the target 104. In various examples, the antenna 112 may emit electromagnetic energy within the radio frequency region or the microwave frequency region of the electromagnetic spectrum. Reflections of the electromagnetic energy from the target 104 are received along a reflected path at the antenna 112 and communicated to the transceiver 114. While illustrated in FIG. 1 as a single component, in various other examples the transceiver 114 may be implemented as a plurality of components, such as a separate transmitter and receiver.

The transceiver 114 generates a charge value (e.g., voltage value) representative of the signal strength (e.g., amplitude) of the received electromagnetic radiation. In various examples, the transceiver 114 receives reflected electromagnetic radiation at the frequency at which the emitted electromagnetic energy passes over the target 104 (referred to as the "re-visit rate"). As illustrated, the transceiver 114 communicates the charge values to the log amplifier 116. While not illustrated in FIG. 1, in various examples the transmitter/receiver electronics may be interposed between the transceiver 114 and the log amplifier 116. Examples of transceiver electronics may include power amplifiers, modulators or demodulators, and/or various other radar antenna base station components.

The log amplifier 116 generates a sequence of samples, indexed by range, based on at least the voltage values provided by the transceiver 114. In particular, samples may be generated based on the received voltage values at the re-visit rate of the antenna 112 and may be representative of the received electromagnetic strength for the corresponding range between the target 104 and the antenna 112. For example, the log amplifier 116 may include one or more digital-to-analog converters that quantize the received voltage values and output integer value samples at the re-visit rate. In various examples, each sample within the sequence is a geometric quantitation (e.g., on a logarithmic scale) of a voltage value provided by the transceiver 114 based on the received electromagnetic energy. That is, in various examples the source of geometric quantitation is a transfer function of the log amplifier 116. As discussed herein, in various instances, amplitudes of received electromagnetic energy rises rapidly as the target 104 approaches the radar antenna system 100 (i.e., the range between the target 104 and the antenna 112 decreases). Accordingly, in various examples the sequence of samples geometrically increases in value relative to a previous sample of the sequence of samples as the target 104 approaches the radar antenna 112.

In various examples, the system interface 106 of the radar antenna system 100 receives the sequence of samples from the log amplifier 116, or the digital-to-analog converter of the log amplifier 116. The system interface 106 may include one or more input device, one or more output devices, or a combination of input and output devices. Based on the received sequence of samples, the interpolation component 108 interpolates a model curve to the sequence of samples. The adaptive filter 110 receives the model curve from the interpolation component 108, compares the model curve to a calibrated curve, and determines a shift between the model curve and the calibrated curve based at least on the comparison. As further described below, the shift may be a shift in a predetermined parameter of the model curve and the calibrated curve, such as a shift in range. Based on the determined shift, the adaptive filter 110 may then detect a RCS of the target 104 by correlating a magnitude of the shift to that RCS.

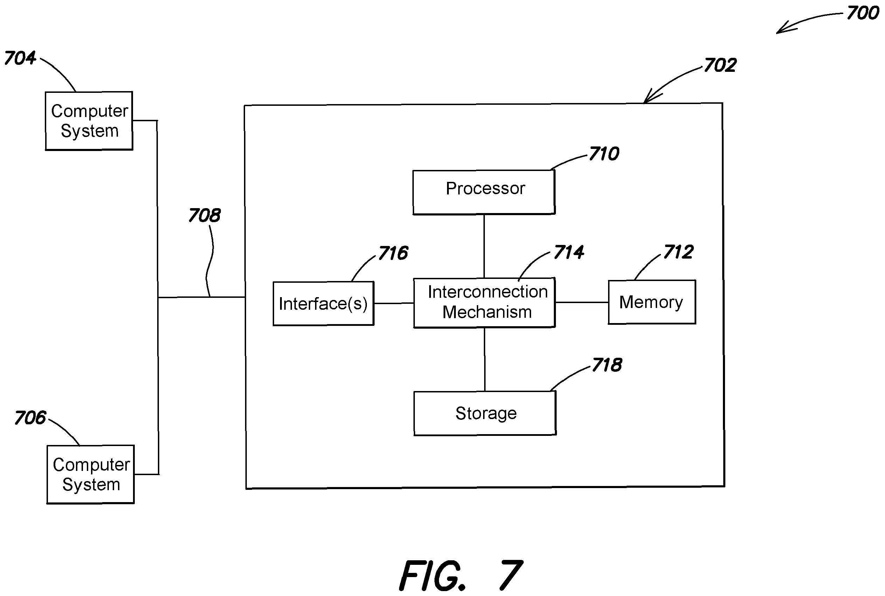

While FIG. 1 illustrates one example, the radar antenna system 100 may be implemented in a variety of ways. In some examples, the radar antenna system 100 may be implemented on a specialized computer system, such as the distributed computer system, or one or more of the computer systems of the distributed computer system, described below with reference to FIG. 7. The computer system may be coupled to other systems, or integrated within other systems, such as radar antenna base station systems. For example, the interpolation component 108 and the adaptive filter 110 may be implemented as software components that are stored within a data storage element of the computer system and executed by a processor. However, in various other examples the radar antenna system 100 may form part of a maritime radar system, an aircraft radar system, or a spacecraft radar system.

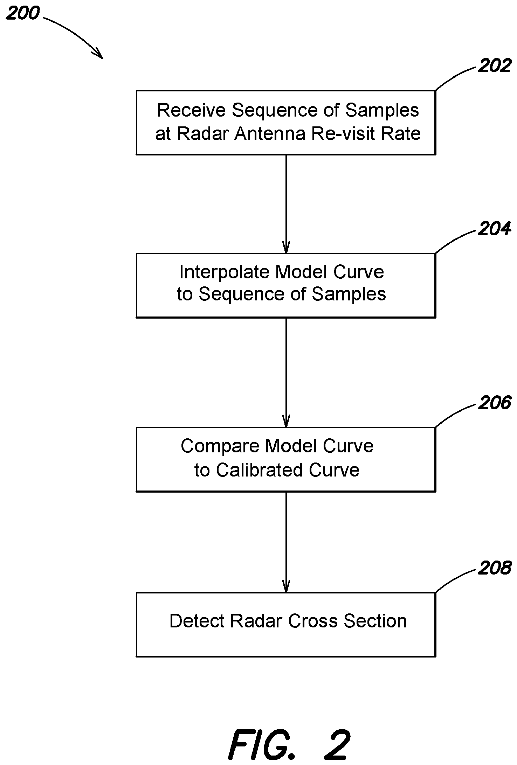

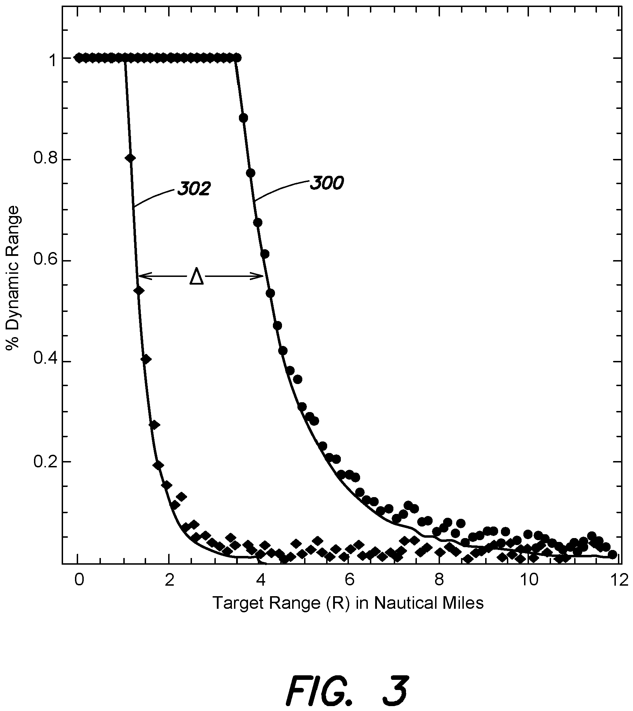

The system interface 106 may be a hardware interface or a software interface component. The software interface component may be implemented on the distributed computer system, or one or more of the computer systems of the distributed computer system, described below with reference to FIG. 7. The system interface 106 allows the radar antenna system 100 to exchange information and communicate with external entities, such as users and other systems. The system interface 106 may exchange data via a network connection using various methods, protocols and standards. Regardless of the implementation, the radar antenna system 100 may perform one or more of the processes for detecting a RCS as described in more detail below with reference to FIG. 2 and FIG. 3.

Turning now to FIG. 2, illustrated is one example of a method 200 for detecting an RCS of a target, such as a target aircraft. Various acts of the method 200 described with reference to FIG. 2 may be performed by the example radar antenna system 100 illustrated in FIG. 1, and the components thereof. Accordingly, FIG. 2 is described with continuing reference to FIG.1. In various examples, the method 200 may include the acts of receiving a sequence of samples at a radar antenna re-visit rate, interpolating a model curve to the sequence of samples, comparing the model curve to a calibrated curve, and detecting a RCS.

As illustrated in FIG. 2, in various examples, the method 200 may include receiving a sequence of samples at a re-visit rate of a radar antenna, the sequence of samples being based at least in part on electromagnetic energy reflected from a target (act 202). In various examples, the sequence of samples are received from a log amplifier (e.g.,

log amplifier 116 illustrated in FIG. 1) and include a geometric quantitation based on one or more charge values generated by the radar antenna based on a signal strength of the received electromagnetic energy. Each sample may be indexed with a range measurement corresponding to that sample and provided by the radar antenna (e.g., antenna 112 illustrated in FIG. 1). Specifically, an increase in value of each sample relative to a previous sample in the sequence may correspond to a decrease in range between the target and the radar antenna, relative to a previous range.

The antenna re-visit rate samples the target, converting a continuous time event into a discrete sequence of samples indexed by range, R. In act 204, the method 200 includes interpolating a model curve to the sequence of samples. As described herein with reference to FIGS. 1 - 3, the act of interpolating a model curve to the sequence of samples may include fitting a function to the discrete sequence of samples. In various examples, the model curve is an ideal model predetermined based at least on the parameter to be detected, which in this instance is RCS. One example of an ideal modeling curve is based on the radar equation,

$$f(R) = f(R; k, \sigma) = \frac{k\sigma}{R^4},$$

where R is a range of the target relative to the radar antenna, $k$ is a scaling factor, and $\sigma$ is RCS. In various examples, the sequence of received samples includes at least a start sample and an end sample. The start sample of the sequence of samples corresponds to a first (i.e., initial) electromagnetic energy signal that is detectable by the radar antenna, and the end sample corresponds to last measurement of the electromagnetic energy. The initial sample may be dependent on the noise performance of the radar antenna and the end sample may be dependent on the saturation point of the radar antenna. As discussed above, in various examples the method 200 includes interpolating each sample within the sequence of samples from the start sample to the end sample, to map the ideal model curve to the entire history of collected electromagnetic energy.

In act 206, the method includes comparing the model curve to a calibrated curve and determining a shift between the model curve and the calibrated curve based at least on the comparison. In various examples, the calibrated curve is based on the same ideal model as the calibrated curve. However, the calibrated curve incorporates various known parameters and a single varied parameter, which in this instance is range, R. For instance, the calibrated curve may be generated according to the radar equation,

$$f(R) = f(R; k, \sigma) = \frac{k\sigma}{R^4},$$

where R is range of a test target relative to the radar antenna, k is a scaling factor, and σ is a predetermined radar cross section. The calibrated curve may be established from a multitude of known data collected over a plurality of previous test runs of a radar antenna system to calculate the predetermined (e.g., known) parameters of the calibrated curve.

Referring to FIG. 3, illustrated is one example plot of a model curve 300 and one example plot of a calibrated curve 302. That is, FIG. 3 illustrates a plot of two example targets as they increase in range relative to a radar antenna (e.g., radar antenna 112 illustrated in FIG. 1). As shown, both the model curve 300 and the calibrated curve 302 are function of range, R, where the model curve 300 is based on the range corresponding to the received sequence of samples and the calibrated curve 302 is based on the range of various previous runs of a radar antenna system to calculate a "calibrated" reference curve. In the illustration of FIG. 3, the horizontal axis represents the target range in nautical miles and the vertical axis represents the percent dynamic range. The percent dynamic range indicates the dynamic range of the corresponding radar antenna system (e.g., the range of signal amplitudes that the system can detect).

Returning to FIG. 2, in act 208, the method 200 includes detecting an RCS of the target based at least in part on the shift between the model curve (e.g., model curve 300 in FIG. 3) and the calibrated curve (e.g., calibrated curve 302 in FIG. 3). That is, in various examples, the method 200 includes determining the RCS of the target based on the shift in range between the model curve and the calibrated curve (shift illustrated as Δ in FIG. 3). In various examples the shift is determined by applying an adaptive filter to the model curve. In one example, applying the adaptive filter includes measuring the shift by a least mean square fit of the calibrated curve to the model curve. As further discussed below, in various examples the method may include automatically executing acts 204, 206, and 208.

For example, in one instance the shift amount may be determined by minimizing the least mean square distance of the discrete sequence of samples as a function of the varied parameter (e.g., "shift" parameter) in the family of modeling functions. The minimization may be accomplished by setting a derivative or gradient of the distance function equal to zero and resolving the resulting equation. As a result of the non-linear nature of the system (e.g., due to the geometric quantization), various iterative solutions may be implemented, such as Newton's Method for finding roots of non-linear equations.

For purposes of illustration, FIG. 3 illustrates a shift, Δ, between a 0 dBm curve (e.g., an example of the calibrated curve 302) and a 20 dBm curve (e.g., an example of the model curve 300). In the example, the RCS of the model curve 300 is determined by the shift in the range relative to the calibrated curve 302. The modeling family for ideal model curve 300 for the calibrated curve 302 and the model curve may include:

$$f(R;\,\Delta,k) = Min\left[1.0, \log\left(1 + \frac{k}{(R+\Delta)^4}\right)\right].$$

As discussed, k is a scaling factor that may be determined over multiple runs with a calibrated target of a known RCS (e.g., σ = 1 m²). The remaining parameter, Δ, is determined by a least mean square fit to the calibrated curve 302:

$$f_1(R;\sigma,k) = Min\left[1.0, \log\left(1 + \frac{k\sigma}{(R)^4}\right)\right],$$

$$f_2(R;\Delta,k) = Min\left[1.0, \log\left(1 + \frac{k}{(R+\Delta)^4}\right)\right].$$

Accordingly, the change in RCS (i.e., by σ in $f_1$) produces an effect similar to a shift in range (i.e., Δ in $f_2$). $R^*$ is used to denote the range at which a calibrated 0 dB target (e.g., corresponding to the example 0 dB calibrated curve) reaches a midpoint of the dynamic range. Such a midpoint range, R , may be determined according to:

$$0.5 = \log\left(1 + \left(\frac{k}{(R^*)^4}\right)\right),$$

which may be rewritten as:

$$R^* = \sqrt[4]{\frac{k}{(e^{0.5} - 1)}}.$$

In various examples, $R^*$ remains fixed for the associated radar antenna system. Accordingly, if the following relationship exists:

$$\frac{k\sigma}{R^4} = \frac{k}{(R+\Delta)^4},$$

then the RCS may be detected according to:

$$\sigma = \left(\frac{R^*}{R^* + \Delta}\right)^4 = \frac{1}{1 + 4\left(\frac{\Delta}{R^*}\right) + 6\left(\frac{\Delta}{R^*}\right)^2 + 4\left(\frac{\Delta}{R^*}\right)^3 + \left(\frac{\Delta}{R^*}\right)^4}.$$

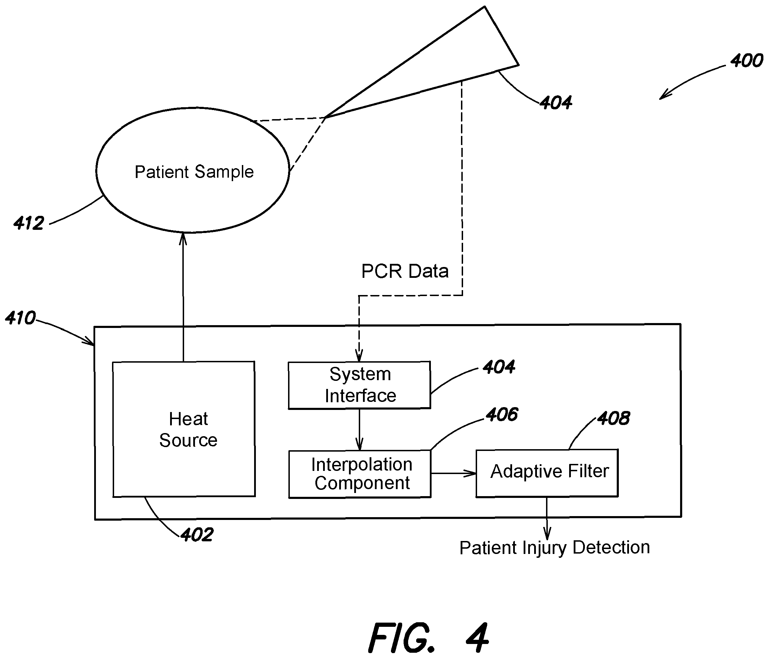

As discussed herein, various aspects and examples are directed to methods and systems for interpolation in systems that execute non-linear quantization routines. While some aspects, such as those described with reference to at least FIGS. 1 - 3 improve current radar antenna systems and RCS detection techniques, other aspects improve current systems and techniques for detecting patient injuries, such as patient injury diagnostic systems and techniques that detect the presence and severity of Traumatic Brain Injury (TBI). One example of a patient injury diagnostic system 400 is illustrated in FIG. 4.

As illustrated in FIG. 4, the patient injury diagnostic system 400 is constructed to detect a patient injury and/or the severity of a patient injury based on a sequence of samples received at a Polymerase Chain Reaction (PCR) cycle rate of a PCR process. As shown, the patient injury diagnostic system 400 may include components configured to execute PCR processes, such as a heat source 402 (e.g., a thermal cycler) and one or more sensors (e.g., the illustrated probe sensor 404). In the illustrated example, the patient injury diagnostic system 400 further includes a system interface 406, an interpolation component 408, and an adaptive filter 410. In some examples the system interface 406, the interpolation component 408, and the adaptive filter 410 may be implemented within the same housing 410 as the heat source 402 and/or the sensor probe 404. However, in various other examples, the patient injury diagnostic system 400 may be located remotely from these components and may be configured to remotely communicate with components configured to execute the PCR processes (e.g., the heat source 402 and the sensor probe 404).

The patient injury diagnostic system 400 is configured to receive a patient sample 412, such as a blood sample, that includes one or more markers. For instance, the patient sample 412 may include one or more blood markers (e.g., cells, proteins, DNA, or RNA), the presence or concentration of which is indicative of a patient injury or the severity of a patient injury. PCR processes may be executed for the patient sample 412 and may include repeatedly replicating the marker (e.g., the blood marker) over one or more cycles of the PCR processes. As further discussed herein, cycles may have integer values may be performed at a frequency referred to as a cycle rate. In various examples, the PCR processes executed by the illustrated patient injury diagnostic system 400, or a system in remote communication with the patient injury diagnostic system 400, include a series of repeated temperature changes. For instance, the heat source 402 may generate and apply a heat cycle to the patient sample 412 for each cycle of the PCR process. The temperature applied, and the duration of the temperature applied, will depend on the particular patient sample and the particular blood marker.

In various examples, a primer that is complementary to the blood marker is added by the patient diagnostic system 400 to the patient sample 412. The primer and the heat cycles cause the blood marker to amplify over the course of the PCR cycles. In particular examples, the concentration of the blood marker may be detected by a fluorescence measurement performed by the probe sensor 404. The probe sensor 404 may include a photodetector or any other suitable optical sensor tuned to detect fluorescence of a biological sample. As discussed above, light emissions of the patient sample 412 (e.g., caused by the reaction of the blood marker with the primer and heat cycles) may correlate to the concentration of the blood marker. Florescence measurements may start in a noise region (e.g., not detectable) and rise rapidly at each cycle of the PCR process until a saturation limit of the probe sensor 404 is reached.

In particular examples, the system interface 404 component may receive a sequence of samples from the probe sensor 404 (shown as PCR data in FIG. 4), or other component of the patient injury diagnostic system 400 coupled to the probe sensor 404, that are correlated to the blood marker concentration in the patient sample 412 and indexed based on the corresponding integer cycle of the PCR process. For example, the system interface 404 may include one or more input device, one or more output devices, or a combination of input and output devices. In various examples, the values of the samples are based on the amplification of the blood concentration and geometrically increase in value relative to a previous sample for each cycle of the PCR process. That is, in various examples, the sequence of samples is a geometric quantitation based on the concentration of the blood marker in the patient sample 412 indexed by a cycle number of the plurality of cycles of the PCR.

While not explicitly illustrated in FIG. 4, in some examples the patient injury diagnostic system 400 may include a quantification component configured to quantize the measured sensor values (e.g., fluorescence) and provide the sequence of samples. In such a situation, the system interface 404 may receive the raw sensor data (PCR data) from the sensor probe 404 and provide the raw sensor data to the quantification component. The interpolation component 406 receives the sequence of samples from the system interface 404 (or the quantification component), and interpolates a model curve to the sequence of samples. The adaptive filter 408 receives the model curve from the interpolation component 406 and compares the model curve to a calibrated curve, and determines a shift between the model curve and the calibrated curve based at least on the comparison. As further described below, the shift may be a shift in a parameter of the model curve and the calibrated curve, such as a shift in PCR cycle. Based on the determined shift, the adaptive filter 408 may detect a patient injury and/or the severity of a patient injury.

While FIG. 4 illustrates one example of the patient injury diagnostic system 400, the patient injury diagnostic system 400 may be implemented in a variety of ways. In some examples, components of the patient injury diagnostic system 400 may be implemented on a specialized computer system, such as the distributed computer system, or one or more the computers system of the distributed computer system, described below with reference to FIG. 7. The computer system may be coupled to other systems, or integrated within other systems. For example, the interpolation component 406, quantification component (not shown), and the adaptive filter 408 may be implemented as software components that are stored within a data storage element of the computer system and executed by a processor.

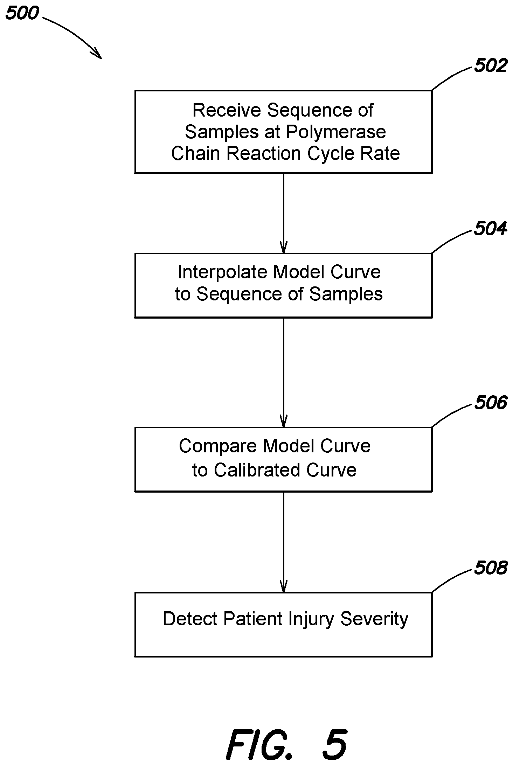

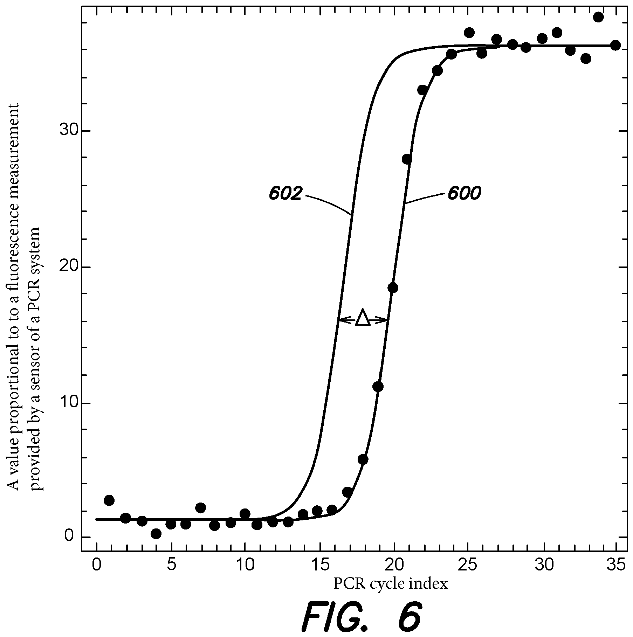

The system interface 404 may be a hardware interface or a software interface component. The software interface component may be implemented on the distributed computer system, or one or more of the computer systems of the distributed computer system, described below with reference to FIG. 7. The system interface 404 allows the patient injury diagnostic system 400 to exchange information and communicate with external entities, such as users and other systems. The system interface 404 may exchange data via a network connection using various methods, protocols and standards. Regardless of the implementation, the patient injury diagnostic system 400 may perform one or more of the processes for detecting the severity or presence of a patient injury as described in more detail below with reference to FIG. 5 and FIG. 6.

Turning now to FIG. 5, illustrated is one example of a method 500 for detecting a patient injury, such as a TBI. Various acts of the method 500 described with reference to FIG. 5 may be performed by the example patient injury diagnostic system 400, and components thereof, illustrated in FIG. 4. Accordingly, FIG. 5 is described with continuing reference to FIG. 5. In various examples, the method 500 may include the acts of receiving a sequence of samples at a PCR cycle rate, interpolating a model curve to the sequence of samples, comparing the model curve to a calibrated curve, and detecting the severity of a patient injury.

As illustrated in FIG. 5, in various examples, the method 500 may include receiving a sequence of samples at a PCR cycle rate, the sequence of samples being based at least in part on a concentration of a blood marker in a patient sample obtained over a plurality of cycles of a PCR (act 502). In various examples, the sequence of samples includes a geometric quantification based on the concentration of the blood marker in the patient sample indexed by a cycle number of the plurality of cycles of the PCR. Specifically, each sample in the sequence of samples may geometrically increase in value relative to a previous sample (e.g., for a previously integer cycle) of the sequence of samples.

In act 504, the method 500 includes interpolating a model curve to the sequence of samples. In various examples, the model curve is an ideal model curve having a plurality of known parameters and a single varying parameter. As described herein with reference to FIGS. 4 - 6, the act of interpolating a model curve to the sequence of samples may include fitting a function to the discrete sequence of quantized samples. In various examples, the model curve is an ideal model predetermined based at least on the measured data, which in this instance is the concentration of the blood marker in the patient sample. One example of the ideal model is based on an equation for modeling the concentration of the blood marker in the patient sample as a function of the PCR cycle,

$$f(C) = (2^C - 2C + 1)X,$$

where C is the cycle number of the PCR process and $X$ is $f(1)$ (i.e., the initial concentration of the blood marker). While in one example, the ideal model is from a family of four-parameter logistic functions (as discussed above), in certain other examples, other families of models may be used, such as those based on a five-parameter logistic function. Such examples may provide a high fidelity match for PCR processes.

In various examples, the sequence of samples includes a start sample and an end sample. The start sample corresponds to an initial sample of the sequence of samples and the end sample corresponds to a last detectable sample of the sequence of samples. For instance, the start sample may correspond to an initial concentration of the blood marker in the patient sample at a first cycle number, and the end sample may correspond to a last concentration of the blood marker at a last cycle number. As discussed herein, in various instances the method 500 includes interpolating the sequence of samples from the start sample to the end sample, to map the ideal model curve to the entire history of samples collected over the full span of PCR cycles.

In act 506, the method 500 includes comparing the model curve to a calibrated curve and determining a shift between the model curve and the calibrated curve based at least on the comparison. In various examples, the calibrated curve is based on the same ideal model as the calibrated curve. However, the calibrated curve incorporates various known parameters and a single varied parameter, which in this instance is the PCR cycle. For instance, the calibrated curve may also be defined according to:

$$f(C) = (2^C - 2C + 1)X,$$

where C is the cycle number of previously collected and calibrated data, and X is f(1) for a known initial concentration of a blood marker in a sample from a health patient. The calibrated curve may be established from a multitude of known data for healthy patients collected over a plurality of test PCR cycles. That is, the calibrated curve may be "calibrated" to be representative of a model for a putative "normal" or healthy population.

Referring to FIG. 6, illustrated is one example plot of a model curve 600 and one example plot of a calibrated curve 602. That is, FIG. 6 illustrates a plot of the interpolated model curve 600 of the sequence of samples received at the PCR cycle rate, and a plot of a calibrated curve 602 for a "healthy" patient based on a similar previously measured PCR cycle rate. In FIG. 6, the horizontal axis represents the PCR cycle index and the vertical axis represents a value proportional to a fluorescence measurement provided by a sensor of a PCR system (e.g., the sensor probe 404 illustrated in FIG. 4). As discussed above, for both curves 600, 602, quantization becomes rapidly coarser until the system reaches saturation. Accordingly, in various examples the method 500 includes detecting a severity of a patient injury based at least in part on the shift between the model curve and the calibrated curve (act 508). That is, in contrast to conventional PCR based approaches, the method 500 includes detecting the severity of a patient injury based at least in part on a shift in the interpolating function as a function of the PCR cycle, rather than the crossing of an interpolating function with a calibrated sensitivity level.

It is appreciated that a change in the blood marker concentration (e.g., the initial blood marker concentration) may directly affect the shift between the model curve and the calibrated curve. The shift in integer cycle between the calibrated curve 602 and the model curve 600 is illustrated as $\Delta$ in FIG. 6. In various examples the shift is determined by applying an adaptive filter to the model curve. In one example, applying the adaptive filter includes measuring the shift by a least mean square fit of the calibrated curve to the model curve. As further discussed above with reference to at least FIG. 2, in various examples the method 500 may include automatically executing acts 504, 506, and 508.

In various examples, the magnitude of the shift may be representative of the presence of an injury or the degree of injury severity. For instance, as the shift grows is magnitude, the likelihood of an injury or the severity of the injury may increase.

As discussed above, in various examples the method 500 may include one or more acts of determining the shift between the model curve and the calibrated curve by measuring the shift by a least mean square fit of the calibrated curve to the model curve (e.g., act 506). For example, for a given family of functions based on PCR cycles, $f(C; C_0)$, where $C$ is the integer cycle and $C_0$ is the initial cycle, and a set of data, $\{(f_C, C): C = 1, 2, ..., N\}$, the method includes determining the parameter $C_0$ to minimize the value function, $V(C_0)$:

$$V(C_0) = \sum_{C=1}^{N} \left(f_C - f(C;\ C_0)\right)^2.$$

For example, method may include finding the value of $C_0$ that makes a derivative of $V(C_0)$ vanish (e.g., an extreme point). For instance, this may be represented as:

$$0 = V'(C_0) = -2\sum_{C=1}^{N} \left((f_C - f(C;\ C_0))\right)\frac{df}{dC_0}.$$

In certain examples, when the derivative is a non-linear function of the parameter, Newton's Method for finding roots of non-linear equations may be used. Newton's method may include an iterative scheme requiring a first "guess" and a criterion for when to stop (i.e., when does the approximation given by iteration sufficiently accurate.) In various embodiments, care is taken to ensure the data is not corrupted in ways to cause Newton's method to fail.

FIG. 7 shows a block diagram of a distributed computer system 700, in which various aspects and functions in accord with the present systems and methods may be practiced. The distributed computer system 700 may include one more computer systems that can be specially configured to perform the functions, operations, and/or processes disclosed herein (e.g., interpolating a model curve, comparting the model curve to a calibrated curve, and/or determining a shift between the model curve and the calibrated curve). For example, as illustrated, the distributed computer system 700 includes three computer systems 702, 704 and 706. As shown, the computer systems 702, 704 and 706 are interconnected by, and may exchange data through, a communication network 708. The network 708 may include any communication network through which computer systems may exchange data. To exchange data via the network 708, the computer systems 702, 704, and 706 and the network 708 may use various methods, protocols and standards including, among others, token ring, Ethernet, Wireless Ethernet, Bluetooth, TCP/IP, UDP, HTTP, FTP, SNMP, SMS, MMS, SS7, JSON, XML, REST, SOAP, CORBA IIOP, RMI, DCOM and Web Services.

Various aspects and functions in accord with the present invention may be implemented as specialized hardware or software executing in one or more computer systems including the computer system 702 shown in FIG. 7. As depicted, the computer system 702 includes a processor 710, a memory 712, a bus 714, an interface 716, and a storage system 718. The processor 710, which may include one or more microprocessors or other types of controllers, can perform a series of instructions that manipulate data. The processor 710 may be, for example, a commercially available processor or controller. As shown, the processor 710 is connected to other system placements, including a memory 712, by the bus 714.

The memory 712 may be used for storing programs and data during operation of the computer system 702. For example, the memory 712 may store ideal model information employed in the processes for interpolating a model curve or comparing the model curve and calibrated curve. Thus, the memory 712 may be a relatively high performance, volatile, random access memory such as a dynamic random access memory (DRAM) or static memory (SRAM). However, the memory 712 may include any device for storing data, such as a disk drive or other non-volatile storage device, such as flash memory or phase-change memory (PCM).

Components of the computer system 702 may be coupled by an interconnection element such as the bus 714. The bus 714 may include one or more physical busses (for example, busses between components that are integrated within a same machine), and may include any communication coupling between system placements including specialized or standard computing bus technologies. Thus, the bus 714 enables communications (for example, data and instructions) to be exchanged between system components of the computer system 702.

Computer system 702 also includes one or more interfaces 716 such as input devices, output devices and combination input/output devices. The interface devices 716 may receive input, provide output, or both. For example, output devices may render information for external presentation. Input devices may accept information from external sources. The interface devices 716 allow the computer system 702 to exchange information and communicate with external entities, such as users and other systems. In some examples, the computer system 702 may exchange information with a radar antenna and/or a PCR based diagnostic system via the interface 716, as discussed above.

Storage system 718 may include a computer-readable and computer-writeable nonvolatile storage medium in which instructions are stored that define a program to be executed by the processor. The instructions may be persistently stored as encoded signals, and the instructions may cause a processor to perform any of the functions described herein. A medium that can be used with various examples may include, for example, optical disk, magnetic disk or flash memory, among others. In operation, the processor 710 or some other controller may cause data to be read from the nonvolatile recording medium into another memory, such as the memory 712, that allows for faster access to the information by the processor 710 than does the storage medium included in the storage system 718. The memory may be located in the storage system 718 or in the memory 712. The processor 710 may manipulate the data within the memory 712, and then copy the data to the medium associated with the storage system 718 after processing is completed.

Various aspects and functions in accord with the present invention may be practiced on one or more computers having different architectures or components than that shown in FIG. 7. For instance, the computer system 702 may include specially-programmed, special-purpose hardware, such as for example, an application-specific integrated circuit (ASIC) tailored to perform a particular operation disclosed herein.

Having described above several aspects of at least one example, it is to be appreciated various alterations, modifications, and improvements will readily occur to those skilled in the art. Such alterations, modifications, and improvements are intended to be part of this disclosure and are intended to be within the scope of the invention. Accordingly, the foregoing description and drawings are by way of example only, and the scope of the invention should be determined from proper construction of the appended claims.

**Claims**

1. A method (200) of detecting radar cross section, the method comprising:

   receiving (202) a sequence of samples at a re-visit rate of a radar antenna (100) the sequence of samples being based at least in part on electromagnetic energy reflected from a target, wherein receiving the sequence of samples includes receiving the sequence of samples from a log amplifier of the radar antenna, the sequence of samples being a geometric quantitation of charge values generated by the radar antenna based on the electromagnetic energy reflected from the target;
   interpolating (204) a model curve to the sequence of samples, wherein each sample of the sequence of samples geometrically increases in value relative to a previous sample of the sequence of samples, wherein the increase in value of each sample relative to a previous sample corresponds to a decrease in a range of the target relative to the radar antenna;
   comparing (206) the model curve to a calibrated curve and determining a shift between the model curve and the calibrated curve based at least on the comparison; and detecting (208) a radar cross section based at least in part on the shift between the model curve and the calibrated curve.

2. The method of claim 1, wherein each of the model curve and the calibrated curve are a function of range, and wherein the shift is a shift in range between the model curve and the calibrated curve.

3. The method of claim 1, wherein the model curve and the calibrated curve are defined according to an ideal model, and wherein the ideal model is:

$$f(R) = f(R; k, \sigma) = \frac{k\sigma}{R^4},$$

wherein R is range, k is a scaling factor, and $\sigma$ is radar cross section.

4. The method of claim 1, wherein determining a shift between the model curve and the calibrated curve includes measuring the shift by a least mean square fit of the calibrated curve to the model curve.

5. The method of claim 1, wherein the sequence of samples includes at least a start sample and an end sample, wherein the start sample corresponds to a first measurement of the electromagnetic energy reflected from the target and the end sample corresponds to a last measurement of the electromagnetic energy reflected from the target.

6. The method of claim 5, wherein interpolating the model curve to the sequence of samples includes interpolating each sample within the sequence of samples from the start sample to the end sample.

7. A computer-readable medium comprising computer executable instructions to cause a computer system (702) that receives information from the radar antenna (100) to execute the steps of any one of claims 1 to 6.

**Patentansprüche**

1. Verfahren (200) zum Detektieren eines Radarquerschnitts, wobei das Verfahren Folgendes umfasst:

Empfangen (202) einer Sequenz von Abtastwerten mit einer Wiederabdeckungsrate einer Radarantenne (100), wobei die Sequenz von Abtastwerten wenigstens teilweise auf elektromagnetischer Energie basiert, die von einem Ziel reflektiert wird, wobei das Empfangen der Sequenz von Abtastwerten Empfangen der Sequenz von Abtastwerten von einem logarithmischen Verstärker der Radarantenne beinhaltet, wobei die Sequenz von Abtastwerten eine geometrische Quantisierung von Ladungswerten ist, die durch die Radarantenne basierend auf der von dem Ziel reflektierten elektromagnetischen Energie erzeugt werden;
Interpolieren (204) einer Modellkurve bezüglich der Sequenz von Abtastwerten, wobei jeder Abtastwert der Sequenz von Abtastwerten bezüglich eines Wertes relativ zu einem vorherigen Abtastwert der Sequenz von Abtastwerten geometrisch zunimmt, wobei die Zunahme des Wertes jedes Abtastwertes relativ zu einem vorherigen Abtastwert einer Abnahme einer Entfernung des Ziels relativ zu der Radarantenne entspricht;
Vergleichen (206) der Modellkurve mit einer kalibrierten Kurve und Bestimmen einer Verschiebung zwischen der Modellkurve und der kalibrierten Kurve basierend auf wenigstens dem Vergleich; und
Detektieren (208) eines Radarquerschnitts basierend wenigstens teilweise auf der Verschiebung zwischen der Modellkurve und der kalibrierten Kurve.

2. Verfahren nach Anspruch 1, wobei sowohl die Modellkurve als auch die kalibrierte Kurve eine Funktion der Entfernung sind und wobei die Verschiebung eine Verschiebung in einem Bereich zwischen der Modellkurve und der kalibrierten Kurve ist.

3. Verfahren nach Anspruch 1, wobei die Modellkurve und die kalibrierte Kurve gemäß einem idealen Modell definiert sind und wobei das ideale Modell Folgendes ist:

$$f(R) = f(R, k, \sigma) = \frac{k\sigma}{R^4},$$

wobei $R$ eine Entfernung ist, $k$ ein Skalierungsfaktor ist und $\sigma$ ein Radarquerschnitt ist.

4. Verfahren nach Anspruch 1, wobei das Bestimmen einer Verschiebung zwischen der Modellkurve und der kalibrierten Kurve Messen der Verschiebung durch einen Fit der kleinsten mittleren Quadrate der kalibrierten Kurve an die Modellkurve beinhaltet.

5. Verfahren nach Anspruch 1, wobei die Sequenz von Abtastwerten wenigstens einen Startabtastwert und einen Endabtastwert beinhaltet, wobei der Startabtastwert einer ersten Messung der von dem Ziel reflektierten elektromagnetischen Energie entspricht und der Endabtastwert einer letzten Messung der von dem Ziel reflektierten elektromagnetischen Energie entspricht.

**6.** Verfahren nach Anspruch 5, wobei das Interpolieren der Modellkurve bezüglich der Sequenz von Abtastwerten Interpolieren jedes Abtastwerts innerhalb der Sequenz von Abtastwerten von dem Startabtastwert zu dem Endabtastwert beinhaltet.

**7.** Computerlesbares Medium, das computerausführbare Anweisungen umfasst, die ein Computersystem (702), das Informationen von der Radarantenne (100) empfängt, zum Ausführen der Schritte nach einem der Ansprüche 1 bis 6 veranlasst.

**Revendications**

**1.** Procédé (200) de détection d'une surface équivalente radar, le procédé comprenant :

la réception (202) d'une séquence d'échantillons à un taux de revisite d'une antenne radar (100), la séquence d'échantillons étant fondée au moins en partie sur l'énergie électromagnétique réfléchie à partir d'une cible, la réception de la séquence d'échantillons incluant la réception de la séquence d'échantillons depuis un amplificateur logarithmique de l'antenne radar, la séquence d'échantillons étant une quantification géométrique de valeurs de charge générées par l'antenne radar sur la base de l'énergie électromagnétique réfléchie par la cible, l'interpolation (204) d'une courbe de modélisation pour la séquence d'échantillons, chaque échantillon de la séquence d'échantillons augmentant géométriquement de valeur par rapport à un échantillon précédent de la séquence d'échantillons, l'augmentation de valeur de chaque échantillon par rapport à un échantillon précédent correspondant à une diminution de la plage de la cible par rapport à l'antenne radar,
la comparaison (206) de la courbe de modélisation à une courbe calibrée et la détermination d'un décalage entre la courbe de modélisation et la courbe calibrée sur la base au moins de la comparaison, et la détection (208) d'une surface équivalente radar sur la base au moins en partie du décalage constaté entre la courbe de modélisation et la courbe calibrée.

**2.** Procédé selon la revendication 1, dans lequel chacune de la courbe de modélisation et de la courbe calibrée est une fonction de la plage, et dans lequel le décalage est un décalage de plage entre la courbe de modélisation et la courbe calibrée.

**3.** Procédé selon la revendication 1, dans lequel la courbe de modélisation et la courbe calibrée sont définies en fonction d'un modèle idéal, le modèle idéal étant :

$$f(R) = f(R; k, \sigma) = \frac{k\sigma}{R^{4'}}$$

dans laquelle R représente la plage, K est un facteur d'échelle et $\sigma$ représente la surface équivalente radar.

**4.** Procédé selon la revendication 1, dans lequel la détermination d'un décalage entre la courbe de modélisation et la courbe calibrée inclut la mesure du décalage par au moins un ajustement quadratique moyen de la courbe calibrée sur la courbe de modélisation.

**5.** Procédé selon la revendication 1, dans lequel la séquence d'échantillons inclut au moins un échantillon de début et un échantillon de fin, l'échantillon de début correspondant à une première mesure de l'énergie électromagnétique réfléchie par la cible et l'échantillon de fin correspondant à la dernière mesure de l'énergie électromagnétique réfléchie par la cible.

**6.** Procédé selon la revendication 5, dans lequel l'interpolation de la courbe de modélisation sur la séquence d'échantillons inclut l'interpolation de chaque échantillon à l'intérieur de la séquence d'échantillons depuis l'échantillon de début jusqu'à l'échantillon de fin.

**7.** Support pouvant être lu par ordinateur comprenant des instructions exécutables par ordinateur permettant d'amener un système informatique (702) qui reçoit des informations de l'antenne radar (100) à exécuter les étapes de l'une quelconque des revendications 1 à 6.

Target

104

Electromagnetic Energy

100

112

102

Transmitter/
Receiver

114

Log Amplifier

116

System
Interface

106

Interpolation
Component

108

Adaptive Filter

110

Radar Cross Section
(RCS)

*FIG. 1*

EP 3 475 725 B1

200

```
┌─────────────────────────────────┐ ─202
│   Receive Sequence of Samples   │
│  at Radar Antenna Re-visit Rate │
└─────────────────────────────────┘
              │
              ▼
┌─────────────────────────────────┐ ─204
│      Interpolate Model Curve     │
│      to Sequence of Samples      │
└─────────────────────────────────┘
              │
              ▼
┌─────────────────────────────────┐ ─206
│        Compare Model Curve       │
│        to Calibrated Curve       │
└─────────────────────────────────┘
              │
              ▼
┌─────────────────────────────────┐ ─208
│     Detect Radar Cross Section   │
└─────────────────────────────────┘
```

# FIG. 2

FIG. 3

FIG. 4

500

502

Receive Sequence of
Samples at Polymerase
Chain Reaction Cycle Rate

504

Interpolate Model Curve
to Sequence of Samples

506

Compare Model Curve
to Calibrated Curve

508

Detect Patient Injury Severity

FIG. 5

*FIG. 6*

FIG. 7

EP 3 475 725 B1

**EP 3 475 725 B1**

**Patent documents cited in the description**

- US 62355686